(19) Europäisches Patentamt
European Patent Office
Office européen des brevets

(11) Veröffentlichungsnummer : **0 042 531**
**B1**

# (12) EUROPÄISCHE PATENTSCHRIFT

(45) Veröffentlichungstag der Patentschrift : **08.02.84**

(51) Int. Cl.³ : **C 07 D209/44**, C 09 B 57/04, C 07 D403/04

(21) Anmeldenummer : **81104425.4**

(22) Anmeldetag : **10.06.81**

## (54) Isoindoleninderivate sowie ihre Verwendung als Zwischenprodukte zur Herstellung von Farbstoffen.

(30) Priorität : **19.06.80 DE 3022839**

(43) Veröffentlichungstag der Anmeldung : **30.12.81 Patentblatt 81/52**

(45) Bekanntmachung des Hinweises auf die Patenterteilung : **08.02.84 Patentblatt 84/06**

(84) Benannte Vertragsstaaten : **CH DE FR GB IT LI**

(56) Entgegenhaltungen :
**DE-A- 1 670 748**
**THE JOURNAL OF ORGANIC CHEMISTRY, Band 44, Nr. 5, 2. März 1979, Seiten 1562, 1563 J.M. McGALL et al.: "Cyanoimine chemistry: New routes to pyrimidinones and (carbonylamino)-imino-propanamides"**
**CHEMISCHE BERICHTE, Jahrgang 106, 1973, Seiten 956-961 W. MERKEL et al.: "Über die Struktur des "4-(Cyanamino)-2-morpholinochinazolins"**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Lorenz, Manfred, Dr.**
**Roggendorfstrasse 51**
**D-5000 Köln 80 (DE)**

EP 0 042 531 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Isoindoleninderivate sowie ihre Verwendung als Zwischenprodukte zur Herstellung von Farbstoffen

Die Erfindung betrifft Verbindungen, die in einer ihrer tautomeren Strukturen der Formel

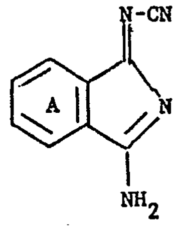

(I)

entsprechen, in der der Ring A durch einen Substituenten aus der Reihe Chlor, Brom, Nitro, $C_1$-$C_6$-Alkyl, insbesondere Methyl und Ethyl, $C_1$-$C_6$-Alkoxy, insbesondere Methoxy und Ethoxy, $C_1$-$C_6$-Alkylsulfonyl, insbesondere Methylsulfonyl, Phenylsulfonyl, Phenyl, Cyan, Amino, Mono-$C_1$-$C_4$-alkylamino, Acetylamino und Di-$C_1$-$C_4$-alkylamino substituiert sein kann.

Die wichtigste Verbindung entspricht in einer ihrer tautomeren Strukturen der Formel

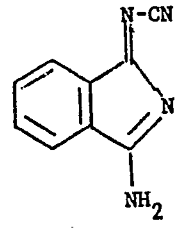

(II)

Die neuen Verbindungen der Formel I können auf verschiedenen Wegen hergestellt werden. Ein bevorzugter Syntheseweg besteht darin, daß man ein gegebenenfalls substituiertes Phthalsäuredinitril in Gegenwart einer basischen Verbindung gegebenenfalls in einem geeigneten Lösungs- oder Verdünnungsmittel mit Cyanamid umsetzt.

Phthalsäuredinitrile, die sich als Ausgangsprodukte für eine derartige Synthese eignen sind z. B. : Phthalsäuredinitril, 3-Nitrophthalsäuredinitril, 4-Nitrophthalsäuredinitril, 4-Chlorphthalsäuredinitril, 4,5-Dichlorphthalsäuredinitril, 4-Aminophthalsäuredinitril, 4-Acetylaminophthalsäuredinitril, 4-Phenylphthalsäuredinitril, 4-Phenylsulfonylphthalsäuredinitril.

Als basische Verbindungen können z. B. Alkali- oder Erdalkalialkoholate bevorzugt niederer Alkohole wie Natriummethylat oder -ethylat, weiterhin Alkali- und Erdalkalimetallhydroxide wie Natrium-, Kalium- und Lithiumhydroxid, Natriumamid und vor allem die Alkalisalze von Cyanamid sowie die Alkalisalze von Verbindungen der Formel I eingesetzt werden.

Geeignete Lösungs- und Verdünnungsmittel sind vorzugsweise niedere Alkohole wie Methanol, Ethanol, Propanol, i-Propanol, Glykole und Glykolderivate wie Ethylenglykol, Glykolmonomethylether, Glykolmonoethylether, Diethylenglykolmonomethyl- und -monoethylether.

Weiterhin kommen infrage : Cyclische oder offenkettige Ether wie Tetrahydrofuran, Dioxan, Ethylenglykoldimethylether, Diethylenglykoldimethylether, Diethylenglykoldiethylether sowie Oligo- und Polyglykole. Gut geeignete Lösungsmittel sind auch Formamid, Methylformamid, Dimethylformamid, Dimethylacetamid, N-Methylpyrrolidon und Dimethylsulfoxid.

Darüber hinaus können viele der üblichen organischen Lösungsmittel eingesetzt werden, sofern sie ein ausreichendes Lösungsvermögen für die umzusetzenden Substanzen aufweisen und nicht zu unerwünschten Nebenreaktionen führen.

Ist diese Löslichkeit für sich zu gering, so können sie auch als zusätzliche Lösungsmittel eingesetzt werden. Solche üblichen organischen Lösungsmittel sind z. B. Benzol, Toluol, Xylol, Chlorbenzol, Dichlorbenzol, Nitrobenzol, Pyridin, Chinolin, Chlortoluol.

Im einzelnen wird die Reaktion so durchgeführt, daß man die Komponenten in beliebiger Reihenfolge mischt, wobei es je nach Umständen, wie Art und Konzentration der eingesetzten Verbindungen, angezeigt sein kann, die Reaktionswärme durch Kühlen abzuführen oder aber die Reaktion durch Erhitzen zu beschleunigen. Im allgemeinen wird man in einem Temperaturbereich arbeiten und die Komponenten so zusammenführen, daß man übermäßiges Kühlen oder Erwärmen vermeidet. Ein derartiger günstiger Temperaturbereich liegt z. B. zwischen ca. 0° und ca. 100 °C, jedoch kann die Reaktion gegebenenfalls auch bei tieferen und höheren Temperaturen ausgeführt werden.

Die zugesetzten basischen Verbindungen dienen als Katalysatoren und müssen demgemäß nicht in stöchiometrischen Mengen eingesetzt werden, jedoch ist es naturgemäß unwirtschaftlich, sehr große Katalysatorenmengen zu verwenden, andererseits aber kann bei zu kleinen Katalysatormengen die Reaktion unwirtschaftlich langsam ablaufen. Günstige Katalysatormengen liegen zwischen etwa 0,1 bis 2-3 Mol, bezogen auf 1 Mol Phthalsäuredinitril.

Das Cyanamid wird zweckmäßigerweise in äquivalenten Mengen oder in einem kleinen Überschuß zugefügt, jedoch sind selbstverständlich größere und kleinere Mengen ebenfalls möglich. Die Verbindungen der Formel I fallen bei der Reaktion ganz oder teilweise als Salze an und können als solche in üblicher Weise, z. B. durch Eindampfen, Ausfällen, Extrahieren oder gegebenenfalls Absaugen isoliert werden.

Werden mit Wasser mischbare Lösungsmittel verwendet, so kann es vorteilhaft sein, nach beendeter Reaktion mit Wasser zu verdünnen und durch Neutralisieren oder Ansäuren das Produkt zu fällen. Man kann in vielen Fällen auch auf eine Isolierung ganz verzichten und die Reaktionsmischungen unmittelbar weiter umsetzen.

Ein zweiter Weg zur Herstellung von Verbindungen der Formel I besteht darin, daß man Verbindungen der Formel III oder Verbindungen der Formel IV mit Cyanamid reagieren läßt.

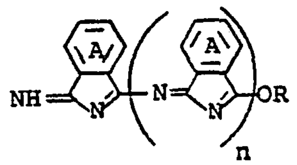
(III)

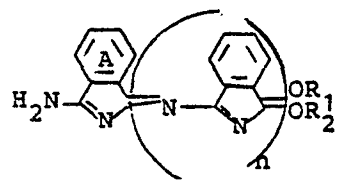
(IV)

In den Formeln III und IV hat A die vorstehend angegebenen Bedeutungen, n bezeichnet 0 oder eine ganze Zahl vorzugsweise 1, 2, 3, 4 oder 5 und R, $R_1$, $R_2$ stehen für Alkyl, vorzugsweise $C_1$-$C_6$-Alkyl, das beispielsweise durch —CN substituiert sein kann, Aralkyl vorzugsweise Benzyl oder Phenethyl, Cycloalkyl vorzugsweise Cyclopentyl und Cyclohexyl, Aryl vorzugsweise Phenyl oder

$$-CH_2-CH_2-(O-CH_2-CH_2-)_m-OH\text{-Reste,}$$

wobei m eine ganze Zahl von 0-40 bezeichnet. Zusammen mit den beiden 0-Atomen und dem C-Atom des Isoindoleninsystems können $R_1$ und $R_2$ auch einen 5- oder 6-gliedrigen heterocyclischen Ring bilden, der beispielsweise durch 1 oder 2 $C_1$-$C_4$-Alkylreste vorzugsweise Methylreste substituiert sein kann.

Verbindungen der Formel III und IV entstehen z. B. bei der Reaktion von Alkoholen oder Glykolen mit Phthalsäuredinitrilen in Gegenwart von basischen Verbindungen. Sie können daher auch bei der in den vorangehenden Abschnitten beschriebenen Umsetzung von Phthalsäuredinitrilen mit Cyanamid in alkoholischen Lösungsmitteln als Zwischenprodukt entstehen. Man kann sie jedoch auch in einen eigenen Reaktionsschritt herstellen und dabei mit oder ohne Zwischenisolierung mit Cyanamid umsetzen, wobei die gleichen Bedingungen wie bei der unmittelbaren Umsetzung von Phthalsäuredinitrilen mit Cyanamid angewendet werden können. Geeignete Verbindungen der Formel III sind z. B. 3-Ethoxy- oder 3-Isopropoxy-1-imino-isoindolenin ; Beispiele für Verbindungen der Formel IV sind 3,3-Dimethoxy-1-imino-isoindolenin und 3,3-Diethoxy-1-amino-isoindolenin. Schließlich können bei der Reaktion von Alkoholen mit Phthalsäuredinitrilen oder mit Amino-imino-isoindolenin unter geeigneten Bedingungen (siehe z. B. Angew. Chemie *68,* 133 (1956)) auch oligomere oder polymere Verbindungen der wahrscheinlichen Formeln III und IV mit $n > 0$ entstehen, die in gleicher Weise verwendet werden können.

Ein weiterer Weg zur Herstellung von Verbindungen der Formel I besteht darin, daß man Verbindungen der Formel

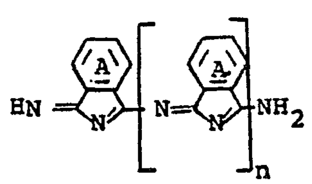
(V)

in der A und n die oben angegebenen Bedeutungen besitzen, mit Cyanamid umsetzt.

Verbindungen der Formel V entstehen z. B. bei der Umsetzung von gegebenenfalls substituierten Phthalsäuredinitrilen mit Ammoniak, wobei im einfachsten Fall ($n = 0$) 1-Amino-3-imino-isoindolenin entsteht.

Die Verbindungen der Formel V reagieren unter ähnlichen Bedingungen mit Cyanamid wie die Phthalodinitrile selbst, wobei bis zu einem Äquivalent Ammoniak freigesetzt wird. Es ist jedoch zweckmäßig, bei Verwendung von Ausgangsverbindungen der Formel V die Reaktionstemperaturen etwas höher zu wählen, um eine ausreichende Reaktionsgeschwindigkeit zu erzielen. Geeignet und ausreichend sind z. B. Temperaturen zwischen 20° und 100 °C.

Die Substanzen der Formel I sind gut kristallisierende Verbindungen, die im IR-Spektrum eine ausgeprägte Nitrilbande bei 2 200 $cm^{-1}$ zeigen.

Die Verbindungen der Formel I eignen sich insbesonders als Ausgangsprodukte für die Herstellung von Farbstoffen. So besteht z. B. ein neuer, vorteilhafter Weg zur Herstellung von Pigmentfarbstoffen darin, daß man Verbindungen der Formel I mit Barbitursäure oder ihren Derivaten der Formel

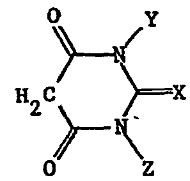

(VI)

in an sich bekannter Weise zu Farbstoffen der Formel VII

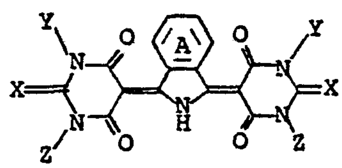

(VII)

kondensiert.

In den Formeln VI und VII stehen :

X für O, S, NH und N—CN und

Y, Z für H, $C_1$-$C_4$-Alkyl, $C_5$-$C_6$-Cycloalkyl oder für einen gegebenenfalls durch Chlor, Brom, $C_1$-$C_6$-Alkyl, $C_1$-$C_6$-Alkoxy, Cyan, Nitro, Carbamoyl, Sulfamoyl, ($C_1$-$C_6$-Alkyl)-carbonylamino, $C_1$-$C_6$-Alkylsulfonyl substituierten Phenylrest. A hat die oben angegebenen Bedeutungen.

Bevorzugte Reaktionskomponente ist die nicht substituierte Barbitursäure.

Die Kondensation kann in Analogie zu den in der DE-OS 2 041 999 angegebenen Verfahren in organischen Lösungsmitteln zweckmäßigerweise in Gegenwart von organischen oder anorganischen Wasserstoff- oder Ansolvosäuren und/oder eines Acylierungmittels im Temperaturbereich von 80-200 °C durchgeführt werden.

Ein besonders vorteilhaftes Verfahren zur Darstellung des Pigments der Formel

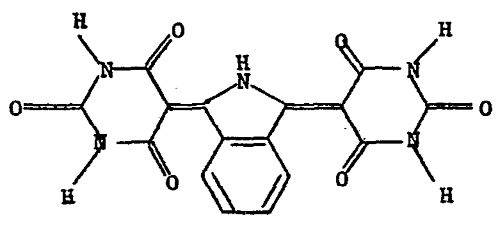

C. I. Pigment Yellow 139

besteht darin, daß man 1-Amino-3-cyanimino-isoindolenin mit Barbitursäure im Verhältnis 1 : 2 Mol kondensiert, wobei die Kondensation vorzugsweise in Wasser im pH-Bereich zwischen 1 und 6 und gegebenenfalls in Gegenwart von oberflächenaktiven Verbindungen bei Temperaturen zwischen 20 und 150 °C durchgeführt wird. Besonders bewährt hat sich eine Arbeitsweise in Analogie zur DE-OS 2 628 409, wobei die Kondensation in Wasser in Gegenwart von Mineralsäuren, aliphatischen und aromatischen Carbonsäuren, aliphatischen und aromatischen Sulfonsäuren oder Gemischen der genannten Säuren durchgeführt wird.

Aus der DE-A 2 628 409 ist ein Verfahren bekannt bei dem 1-Amino-3-imino-isoindolenin mit Barbitursäure zu Pigmentfarbstoffen kondensiert wird. Bei Verwendung des erfindungsgemäßen 1-Amino-3-cyanimino-isoindolenins an Stelle von 1-Amino-3-imino-isoindolenin in der Kondensationsreaktion mit Barbitursäure wird, unter ansonst gleichen Bedingungen, eine höhere Pigmentausbeute erzielt.

In den folgenden Beispielen werden unter « Teile » Gewichtsteile verstanden. Diese verhalten sich zu den Raumteilen wie g zu ml. Die Temperaturangaben erfolgen in °C.

## Beispiel 1

33,6 Teile Mononatriumcyanamid werden mit 150 Raumteilen Methanol verrührt. In ca. 2-3 Stunden trägt man bei 30-35° 64 Teile Phthalsäuredinitril ein und rührt über Nacht bei Raumtemperatur. Das Reaktionsgemisch wird mit 300 Raumteilen Eiswasser verdünnt und die Lösung durch Zutropfen von Salzsäure oder Essigsäure neutralisiert. Es bildet sich ein Niederschlag, der nach 15 Minuten abgesaugt, mit Wasser gewaschen und bei 60° im Vakuum getrocknet wird.

Man erhält 86 Teile einer farblosen, kristallinen Substanz, die bis 247-250° schmilzt und deren Analyse eine empirische Formel von $C_9H_6N_4 \cdot 1/2\ H_2O$ ergibt.

Analyse :

berechnet : C 60,3, H 3,9, N 31,3

gefunden : C 60,2, H 4,1, N 31,2

Ein vergleichbares Ergebnis wird erzielt, wenn man Methanol in Beispiel 1 durch Glykol oder Methylglykol ersetzt.

## Beispiel 2

91 Teile Dinatriumcyanamid werden in 800 Raumteile Methanol eingestreut, wobei man durch Kühlen die Temperatur bei 30-35° hält. Anschließend trägt man in ca. 1 Stunde in kleinen Portionen insgesamt 128 Teile Phthalsäuredinitril so ein, daß die Temperatur 60° nicht übersteigt, und rührt noch 1 Stunde nach. Anschließend gießt man die Reaktionsmischung in 1 000 Raumteile Eiswasser, klärt und neutralisiert das Filtrat durch Zutropfen von 210 Raumteilen 10 N Salzsäure. Man erhält einen Niederschlag, der wie in Beispiel 1 beschrieben, isoliert und getrocknet wird. Die Ausbeute beträgt 160 Teile.

Das Produkt zeigt die gleichen Eigenschaften, wie in Beispiel 1 beschrieben.

An Stelle von Methanol kann in Beispiel 2 mit vergleichbaren Resultaten Ethanol eingesetzt werden.

## Beispiel 3

64 Teile Phthalsäuredinitril und 200 Raumteile Methanol werden verrührt und 46 Teile einer 30 %igen Natriummethylatlösung zugegeben. Die Mischung wird 30 Minuten auf 55-60° erhitzt. Man erhält eine dunkel gefärbte Lösung von mono- und oligomeren Methoxy-amino-isoidoleninen von unbekannter Zusammensetzung. In diese Mischung streut man in ca. 30 Minuten 21,5 Teile Cyanamid ein und rührt noch 1 Stunde bei 60°. Die erhaltene Lösung gibt man in 1 000 Raumteile Wasser und neutralisiert mit 16 Raumteilen Essigsäure. Arbeitet man wie in Beispiel 1 beschrieben auf, so erhält man 77,8 Teile 1-Amino-3-cyanimino-isoindolenin.

## Beispiel 4

Verfährt man wie in Beispiel 3 beschrieben, verwendet aber lediglich 23 Teile Natriummethylatlösung, so erhält man 1-Amino-3-cyanimino-isoindolenin mit gleicher Ausbeute wie in Beispiel 3 angegeben.

## Beispiel 5

128 Teile Phthalsäuredinitril werden unter Kühlung bei 25° in eine Lösung von 12,5 Teilen Natrium in 1 000 Raumteilen Ethanol eingetragen und 1,5 Stunden nachgerührt. Zu dieser so erhaltenen Suspension von 1-Amino-3,3-diethoxy-isoindolenin gibt man 43 Teile Cyanamid und erhitzt anschließend 1 Stunde auf 60°. Die Aufarbeitung ergibt 170 Teile 1-Amino-3-cyanimino-isoindolenin mit den gleichen Eigenschaften wie in Beispiel 1.

## Beispiel 6

73 Teile 1-Amino-3-imino-isoindolenin werden mit 500 Raumteilen Methanol vermischt, 33,6 Teile Mononatriumcyanamid zugegeben und 2 Stunden unter Rückfluß erhitzt, wobei Ammoniak entweicht. Die Lösung wird in 1 000 Raumteile Eiswasser eingegossen und wie in Beispiel 1 das Amino-cyaniminoisoindolenin isoliert. Man erhält eine Ausbeute von 68 Teilen.

## Beispiel 7

63,2 Teile Mononatriumcyanamid werden mit 1 500 Raumteilen Methanol verrührt und in ca. 1 Stunde 163 Teile 4-Chlor-phthalsäuredinitril eingetragen. Die Mischung wird über Nacht bei Raumtemperatur gerührt, danach mit 1 000 Raumteilen Wasser verdünnt, mit 100 Raumteilen Essigsäure angesäuert und 2 Stunden nachgerührt.

Der sich bildende Niederschlag wird abgesaugt und mit Methanol und Wasser gewaschen. Man erhält 163 Teile 4- oder 5-Chlor-1-amino-3-cyaniminoisoindolenin mit einem Schmelzpunkt von 253-256 °C. Die Substanz ist im Dünnschichtchromatogramm weitgehend einheitlich.

Analyse :

berechnet : C 52,8, H 2,5, N 27,4

gefunden : C 52,5, H 2,6, N 26,9

## Beispiel 8

Verfährt man wie in Beispiel 7, setzt jedoch an Stelle von Chlorphthalsäuredinitril 173 Teile 4-Nitrophthalsäuredinitril ein, so erhält man 204 Teile 4- oder 5-Nitro-amino-3-cyanimino-isoindolenin. Schmelzpunkt : 244-251 °C.

Analyse :
berechnet : C 50,2, H 2,3, N 32,6
gefunden : C 49,9, H 2,6, N 32,2

Beispiel 9

300 Raumteile Methanol und 37 Teile 4-Acetylaminophthalsäuredinitril werden verrührt, 13,5 Teile Mononatriumcyanamid eingestreut, über Nacht bei Raumtemperatur gerührt und noch 1 Stunde unter Rückfluß erhitzt. Danach wird noch in der Siedehitze durch Zutropfen von 12 Raumteilen Essigsäure neutralisiert. Die Suspension wird mit 300 Raumteilen Eiswasser versetzt, abgesaugt und mit Wasser gewaschen. Man erhält 39,5 Teile 4- oder 5-Acetylamino-1-amino-cyaniminoisoindolenin. Die Substanz zeigt keinen definierten Schmelzpunkt.

Beispiel 10

27,2 Teile Barbitursäure, 500 Raumteile Wasser und 7,7 Raumteile Ameisensäure werden verrührt und 17,9 Teile 1-Amino-3-cyanimino-barbitursäure, hergestellt nach Beispiel 1, eingestreut. Der Ansatz wird 2 Stunden bei 60° und 4 Stunden unter Rückfluß gerührt. Danach wird der Niederschlag abgesaugt und mit 200 Raumteilen Wasser von 80° gewaschen. Nach dem Trocknen bei 70° erhält man 35,5 Teile eines gelben Pigmentes der Formel

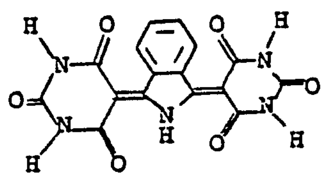

Analyse :
berechnet : C 52,3, H 2,5, N 19,1
gefunden : C 52,0, H 2,6, N 19,1

Beispiel 11

Verfährt man wie bei Beispiel 10, setzt jedoch dem Reaktionsansatz zusätzlich 3 Teile eines Ethylenoxyd-Propylenoxyd-Blockpolymersates mit einem Molekulargewicht von ca. 3 000 zu, so erhält man 36,4 Teile des gleichen Pigmentes, jedoch in einer leichter dispergierbaren Form.

Beispiel 12

Verfährt man wie bei Beispiel 10 beschrieben, setzt jedoch an Stelle von Barbitursäure eine analoge Menge N,N'-Dimethylbarbitursäure ein, so isoliert man 38,9 Teile eines gelben Farbstoffes der Formel

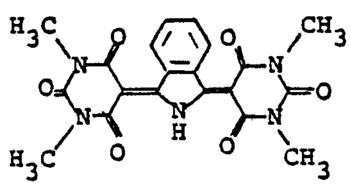

Analyse :
berechnet : C 56,7, H 4,0, N 16,5
gefunden : C 56,6, H 3,9, N 16,4

Beispiel 13

Setzt man an Stelle von Barbitursäure in Beispiel 10 eine entsprechende Menge 2-Thiobarbitursäure ein und verfährt im übrigen wie in Beispiel 10 beschrieben, so erhält man 27,3 Teile des roten Farbstoffes der Formel

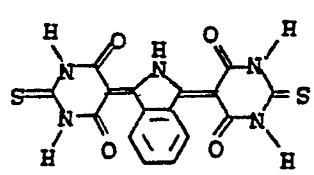

**Ansprüche**

1. Verbindungen, die in einer ihrer tautomeren Strukturen der Formel

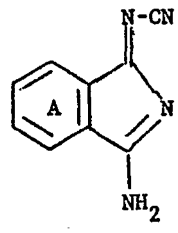

entsprechen, bei denen der Ring A durch einen Substituenten aus der Reihe Chlor, Brom, Nitro, $C_1$-$C_6$-Alkyl, $C_1$-$C_6$-Alkoxy, $C_1$-$C_6$-Alkylsulfonyl, Phenylsulfonyl, Phenyl, Cyan, Amino, Mono-$C_1$-$C_4$-alkylamino, Di-$C_1$-$C_4$-alkylamino, Acetylamino substituiert sein kann.

2. Verbindung, die in einer ihrer tautomeren Strukturen der Formel

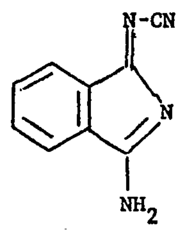

entspricht.

3. Verfahren zur Herstellung von Farbstoffen der Formel

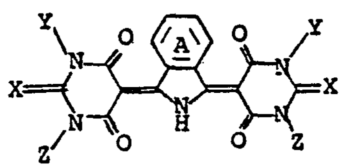

in der

X für O, S, NH und N—CN und

Y, Z für H, $C_1$-$C_4$-Alkyl, $C_5$-$C_6$-Cycloalkyl oder für einen Phenylrest, der durch Chlor, Brom, $C_1$-$C_6$-Alkyl, $C_1$-$C_6$-Alkoxy, Cyan, Nitro, Carbamoyl, Sulfamoyl, ($C_1$-$C_6$-Alkyl)-carbonylamino oder $C_1$-$C_6$-Alkylsulfonyl substituiert sein kann, stehen und bei denen der Ring

A einen Substituenten aus der Reihe Chlor, Brom, Nitro, $C_1$-$C_6$-Alkyl, $C_1$-$C_6$-Alkoxy, $C_1$-$C_6$-Alkylsulfonyl, Phenylsulfonyl, Phenyl, Cyan, Amino, Mono-$C_1$-$C_4$-alkylamino, Di-$C_1$-$C_4$-alkylamino, Acetylamino tragen kann, dadurch gekennzeichnet, daß man Verbindungen, die in einer ihrer tautomeren Strukturen der Formel

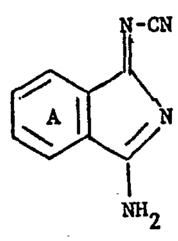

entsprechen, wobei A die oben angegebene Bedeutung hat, mit Barbitursäure oder ihren Derivaten der Formel

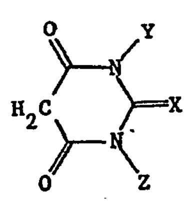

in der X, Y, Z die oben angegebenen Bedeutungen besitzen, in an sich bekannter Weise kondensiert.

4. Verfahren zur Herstellung des Pigments der Formel

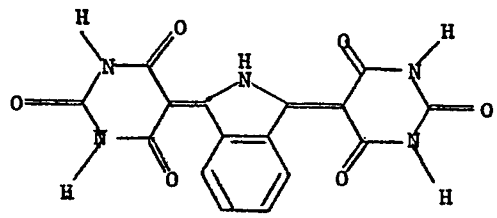

dadurch gekennzeichnet, daß man 1-Amino-3-cyaniminoisoindolenin mit Barbitursäure im Verhältnis 1 : 2 Mol kondensiert, wobei die Kondensation vorzugsweise in Wasser im pH-Bereich zwischen 1 und 6 und gegebenenfalls in Gegenwart von oberflächenaktiven Verbindungen bei Temperaturen zwischen 20 und 150 °C durchgeführt wird.

**Claims**

1. Compounds which, in one of their tautomeric structures, correspond to the formula

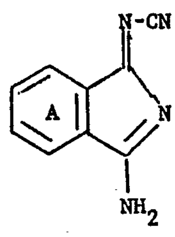

in which the ring A can be substituted by one substituent from the series comprising chlorine, bromine, nitro, $C_1$-$C_6$-alkyl, $C_1$-$C_6$-alkoxy, $C_1$-$C_6$-alkylsulphonyl, phenylsulphonyl, phenyl, cyano, amino, mono-$C_1$-$C_4$-alkylamino, di-$C_1$-$C_4$-alkylamino or acetylamino.

2. Compound which, in one of its tautomeric structures, corresponds to the formula

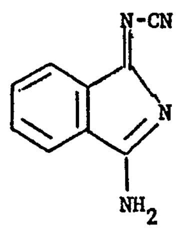

3. Process for the preparation of dyestuffs of the formula

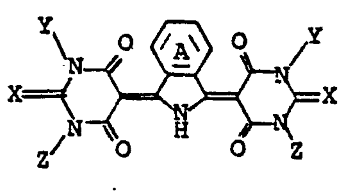

in which

X represents O, S, NH or N—CN and

Y and Z represent H, $C_1$-$C_4$-alkyl, $C_5$-$C_6$-cycloalkyl or a phenyl radical which can be substituted by chlorine, bromine, $C_1$-$C_6$-alkyl, $C_1$-$C_6$-alkoxy, cyano, nitro, carbamoyl, sulphamoyl, ($C_1$-$C_6$-alkyl)-carbonylamino or $C_1$-$C_6$-alkylsulphonyl and in which the ring

A can carry a substituent from the series comprising chlorine, bromine, nitro, $C_1$-$C_6$-alkyl, $C_1$-$C_6$-alkoxy, $C_1$-$C_6$-alkylsulphonyl, phenylsulphonyl, phenyl, cyano, amino, mono-$C_1$-$C_4$-alkylamino, di-$C_1$-$C_4$-alkylamino and acetylamino, characterised in that compounds which, in one of their tautomeric structures, correspond to the formula

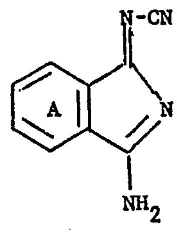

wherein A has the meaning indicated above, are subjected to a condensation reaction with barbituric acid or derivatives thereof of the formula

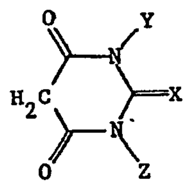

in which X, Y and Z have the meanings indicated above, in a manner which is in itself known.

4. Process for the preparation of the pigment of the formula

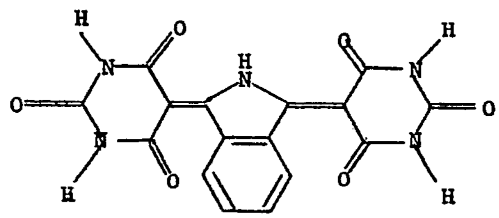

characterised in that 1-amino-3-cyaniminoisoindolenine is subjected to a condensation reaction with barbituric acid in a molar ratio of 1 : 2, the condensation preferably being carried out in water in the pH range between 1 and 6, and if appropriate in the presence of surface-active compounds, at temperatures between 20 and 150 °C.

**Revendications**

1. Composés répondant, dans l'une de leurs structures tautomères, à la formule

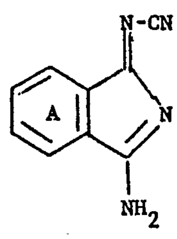

dans laquelle le cycle A peut porter un substituant de la classe chlore, brome, nitro, alkyle en $C_1$-$C_6$, alcoxy en $C_1$-$C_6$, alkylsulfonyle en $C_1$-$C_6$, phénylsulfonyle, phényle, cyano, amino, monoalkylamino en $C_1$-$C_4$, di-(alkyle en $C_1$-$C_4$)-amino, acétylamino.

2. Composé répondant, dans l'une de ses structures tautomères, à la formule

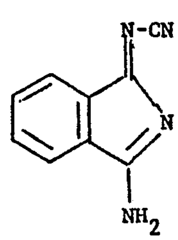

3. Procédé de préparation des colorants de formule

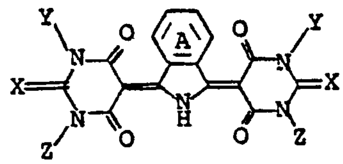

dans laquelle

X représente O, S, NH et N—CN et

Y, Z représentent H, un groupe alkyle en $C_1$-$C_4$, cycloalkyle en $C_5$-$C_6$ ou un reste phényle qui peut être substitué par le chlore, le brome, des groupes alkyle en $C_1$-$C_6$, alcoxy en $C_1$-$C_6$, cyano, nitro, carbamoyle, sulfamoyle, (alkyle en $C_1$-$C_6$)-carbonylamino ou alkylsulfonyle en $C_1$-$C_6$ et dans laquelle le cycle

A peut porter un substituant de la classe chlore, brome, nitro, alkyle en $C_1$-$C_6$, alcoxy en $C_1$-$C_6$, alkylsulfonyle en $C_1$-$C_6$, phénylsulfonyle, phényle, cyano, amino, monoalkylamino en $C_1$-$C_4$, di-(alkyle en $C_1$-$C_4$)-amino, acétylamino, caractérisé en ce que l'on condense de manière connue en soi des composés qui, dans l'une de leurs structures tautomères, répondent à la formule

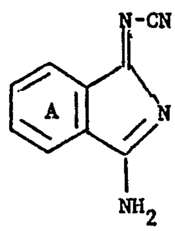

dans laquelle A a la signification indiquée ci-dessus, avec l'acide barbiturique et ses dérivés de formule

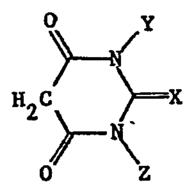

dans laquelle X, Y, Z ont les significations indiquées ci-dessus.

4. Procédé de préparation du pigment de formule

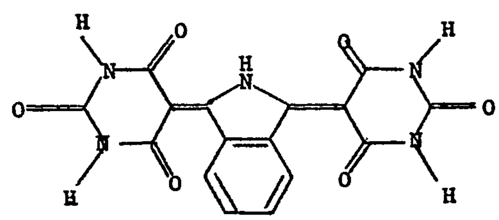

caractérisé en ce que l'on condense la 1-amino-3-cyanimino-isoindolénine avec l'acide barbiturique dans un rapport molaire de 1 : 2, la condensation étant de préférence effectuée dans l'eau dans l'intervalle de pH de 1 à 6 et éventuellement en présence de composés tensioactifs à des températures de 20 à 150 °C.